# EUROPEAN PATENT SPECIFICATION

(11) **EP 1 418 860 B1**
(45) Date of publication and mention of the grant of the patent: **23.04.2008**
(21) Application number: 02767385.4
(22) Date of filing: 15.08.2002
(51) Int. Cl.: A61C 19/06

(54) **ORAL CARE SUBSTANCE DELIVERY STRIP**
STREIFEN ZUR ABGABE EINES MUNDPFLEGEWIRKSTOFFES
BANDE POUR DELIVRER UN PRODUIT DE SOINS BUCCO-DENTAIRE

(30) Priority: 17.08.2001 GB 0120136; 17.08.2001 GB 0120144; 16.01.2002 GB 0200871
(43) Date of publication of application: 19.05.2004
(73) Proprietor: SMITHKLINE BEECHAM PLC, Brentford, Middlesex TW8 9GS (GB)
(72) Inventor: JACKSON, Graham, Surrey KT13 0DE (GB); JONES, Stephen, Surrey KT13 0DE (GB); MACLEOD, Andrew, Hertfordshire SG8 6DP (GB); NOBLE, Michael, Hertfordshire SG8 6DP (GB); WOOD, Timothy, Hertfordshire SG8 6DP (GB)
(74) Representative: Walker, Ralph Francis
(86) International application number: PCT/EP2002/009121
(87) International publication number: WO 2003/015656

(56) References cited:
- EP-A- 0 862 903
- EP-A- 1 088 527
- WO-A-95/16488
- US-A- 2 257 709
- US-A- 5 061 258
- US-A- 5 575 654
- US-A- 5 989 569
- US-A- 6 136 297

## Description

The present invention relates to a device for the delivery of an oral care substance or composition to oral surfaces including teeth, gingival and mucosal tissues.

Delivery devices are known for delivering an oral healthcare substance to the surface of a tooth and oral tissue, comprising a strip of a flexible film on a surface of which is an oral healthcare substance which also acts as an adhesive to attach the device to a tooth surface. See for example WO-A-98/55044, US-A-5 879 691, US-A-5 894 017, WO-A-98/55079, WO-A-95/16488. US-A-6 045 811, WO-A-00/07518 and US-A 2 835 628.

US-A-5 989 569 for example discloses strips of plastically deformable material on which there is an oral healthcare substance to be applied to the teeth.

The problem with these devices is that they are difficult to handle and unpleasant in use. From the consumer perspective, primary importance would be a low cost, non-bulky oral care delivery device that is both easy, effective and unobtrusive in use.

It is an object of this invention to provide alternative devices for delivering an oral healthcare substance to the surface of a tooth. Known devices in which the oral healthcare substance also provides an adhesive function suffer from the disadvantage that for example compatible adhesive and oral healthcare substances must be used, and the present invention aims to alleviate this problem.

According to this invention a delivery device for delivering an oral healthcare substance to the oral surfaces of the teeth, gingival and/or mucosal tissues is provided according to claim 1.

By separation of the adhesive function and oral healthcare substance, greater flexibility of construction options are made available.

The strip of an orally acceptable flexible material may be any material, natural or synthetic, which can be applied to the surface of a tooth by a user, adapted by pressure, e.g a pinching action, to the contours of the surface of a tooth, and subsequently easily removed by the user. The strip should be flexible enough to enable such adaptation to the tooth surface.

This adaptation may be via permanent deformation i.e. in which the strip undergoes plastic deformation with little or no tendency to return to its original shape after adaptation to a tooth surface. The strip comprises a layer of plastically deformable material. By plastically deformable it is meant that the material, at least when in the form of the strip, may be easily deformed by the user using finger or hand pressure, below or at body temperature, so as to fit the device to the overall shape of the user's teeth, preferably also being capable of being deformed into the gaps between the user's teeth. Typically a plastically deformable device should be plastically deformable under the application of a pressure of less than 250,000 pascals, e.g. to allow the strip to be deformed to fit the contours of the users teeth and/or oral tissues. Suitable plastically deformable materials include waxes, in particular dental waxes, e.g. of the known type suitable for making casts of the shape of teeth. The plastically deformable material preferably comprises such a wax. Such a plastically deformable material may provide the adhesive function by means of the material being plastically deformed to closely conform to the shape of the teeth and to the spaces between the teeth, and thereby gripping the teeth independent of any adhesive function provided or not provided by the oral healthcare substance. The gripping action provided by such a plastically deformable material may by by friction between the material and the surfaces of the teeth and/or oral tissues to which it is applied, and/or by the "deadfold" action as the material accomodates to the contours of the surfaces and fits into surface concavities etc. to thereby grip.

The strip material should be compatible with the oral cavity and comfortable for the user. The material may comprise a polymeric film or a fabric material, woven or non-woven. The material may be transparent or opaque, or may be coloured to be visually unobtrusive, or alternatively noticeable when in the mouth, dental waxes as mentioned above being available in a variety of grades and colours such as white, blue and pink. The material may have matter printed thereon, e.g. a manufacturer's or retailer's logo, or other visible symbol. Alternately or additionally the material of the strip may contain a colourant, for example titanium dioxide to impart a bright whiteness to the strip material.

To at least one surface of the strip there is attached a carrier layer of an absorbent material. Such an absorbent material is preferably a fabric, woven or non-woven, preferably non woven. A suitable non-woven fabric is for example, polypropylene, viscose or a polypropylene-viscose blend. The absorbent material may occupy the whole of the said at least one surface of the strip.

The strip material may for example be textured, to provide a surface more able to carry the substance or for user comfort etc. For example the strip material may have wells or dimples in its surface, or may have a sponge-like surface, to contain the substance and/or an adhesive material. The strip material may be hydrophilic, and for example may be a hydrogel polymer, of generally known type. Alternatively the strip material may be hydrophobic. For example a multiple layered strip may comprise one or more hydrophilic layer and one or more hydrophobic layer. The strip material, or the material of one or more of the layers of a multiple layer strip, may be biodegradable in the mouth environment. Suitable film materials are for example the cellulose or polyethylene based materials disclosed respectively in US-A-2,835,628 and WO-A-00/07518, hydroxypropylmethylcelluloses, and fluorinated polymers such as PTFE.

The shape and size of the strip may be such that it can be applied to either a single tooth, for example being of a shape and size corresponding to a surface of an individual tooth, or preferably to a plurality of teeth simultaneously, suitably therefore being an elongate strip of a length corresponding generally to the length of a plurality of teeth to which it is to be applied, and a width corresponding generally to the height of the teeth. Alternatively and preferably the width of the strip may be greater than the height of the teeth to enable the strip to be bent or folder over the crown and behind the teeth to contact the rear surfaces of the teeth.

The term "oral healthcare substance" as used herein includes curative, prophylactic and cosmetic active substances or compositions thereof. Examples of the oral conditions these substances may address include, but are not limited to one or more of, appearance and structural changes to teeth, whitening, stain bleaching, stain removal, plaque removal, tartar removal, cavity prevention and treatment, inflamed and/or bleeding gums, mucosal wounds, lesions, ulcers, aphthous ulcers, cold sores, tooth abscesses, tooth and/or gum pain, tooth sensitivity (e.g. to temperature changes), and the elimination of mouth malodour resulting from the conditions above and other causes such as microbial proliferation.

Suitable oral care actives include any substance that is generally considered as safe for use in the oral cavity and that provides a change to the overall health of the oral cavity. The level of oral care substance in the present invention may generally be from about 0.01 % to about 40 %, preferably from about 0.1 % to 20%.

Oral care substances of the present invention may include many of the actives previously disclosed in the art. The following is a non all-inclusive list of oral care actives that may be used in the present invention.

Teeth whitening actives may be included in the oral care substance of the present invention. The actives suitable for whitening are selected from the group consisting of peroxides, metal chlorites, perborates, percarbonates, peroxyacids, and combinations thereof. Suitable peroxide compounds include: hydrogen peroxide, calcium peroxide, carbamide peroxide, and mixtures thereof. A preferred peroxide is hydrogen peroxide. Suitable metal chlorites include calcium chlorite, barium chlorite, magnesium chlorite, lithium chlorite, sodium chlorite and potassium chlorite. Additional whitening actives may be hypochlorite and chlorine dioxide. A preferred chlorite is sodium chlorite.

Anti-tartar agents that are known for use in dental care products includes phosphates. Phosphates include pyrophosphates, polyphosphates, polyphosphonates and mixtures thereof. Pyrophosphates are among the best known for use in dental care products. Pyrophosphate ions delivered to the teeth derive from pyrophosphate salts. The pryophosphate salts useful in the present compositions include the dialkali metal pyrophosphate salts, tetra-alkali metal pyrophosphate salts, and mixtures thereof. Disodium dihydrogen pyrophosphate (Na₂H₂P₂O₇), tetrasodium pyrophosphate (Na₄P₂O₇), and tetrapotassium pyrophosphate. (K₄P₂O₇) in their unhydrated as well as hydrated forms are preferred. Anticalculus phosphates include potassium and sodium pyrophosphates; sodium tripolyphosphate; diphosphonates, such as ethane-1-hydroxy-1,1-diphosphonate, 1-azacycloheptane-1,1-diphosphonate, and linear alkyl diphosphonates; linear carboxylic acids and sodium and zinc citrate.

Agents may be used in place of or in combination with the pyrophosphate salt include materials as synthetic anionic polymers including polyacrylates and copolymers of maleic anhydride or acid and methyl vinyl ether (e.g. Gantrez™), as described, for example, in U.S. Pat. No. 54,627,977, to Gaffar et al, as well as e.g. polyamino propane sulfonic acid (AMPS), zinc citrate trihydrate, polyphosphates (e.g. tripolyphosphate; hexametaphosphate), diphosphonates (e.g. BHDP, AHP), polypeptides (such as polyaspartic and polyglutamic acids), and mixtures thereof.

Fluoride ion sources are well known for use in oral care compositions as anticaries agents. Fluoride ions are included in many oral care compositions for this purpose, and similarly may be incorporated in the invention in the same way.

Antimicrobial agents can also be present in the oral care compositions or substances of the present invention. Such agents may include, but are not limited to, 5-chloro-2-(2,4-dichlorophenoxy)-phenol, commonly referred to as triclosan, chlorhexidine, alexidine, hexetidine, sanguinarine, benzalkonium chloride, salicylamide, domiphen bromide, cetylpyridium chloride (CPC), tetradecyl-pyridinium chloride (TPC); N-tetradecyl-4-ethyl lpyridinium chloride (TDEPC); octenidine; delmopinol, octapinol, and other piperidino derivatives, niacin preparations; zinc/stannous ion agents; antibiotics such as AUGMENTIN, amoxyicillin, tetracycline, doxycyline, minocycline, and metronidazole; and analogs and salts of the above antimicrobial antiplaque agents.

Anti-inflammatory agents can also be present in the oral care substances or compositions of the present invention. Such agents may include, but are not limited to, non-steroidal anti-inflammatory agents or NSAIDs, such as ketorolac, flurbiprofen, ibuprofen, naproxen, indomethacin, aspirin, ketoprofen, piroxicam and meclofenamic acid.

Nutrients may improve the condition of the oral cavity and can be included in the oral care substances or compositions of the present invention. Examples of nutrients include minerals, vitamins, oral nutritional supplements, enteral nutritional supplements, and mixtures thereof.

An individual enzyme or combination of several compatible enzymes can also be included in the oral care substance or composition of the present invention. Enzymes are biological catalysts of chemical reactions in living devices. Enzymes combine with the substrates on which they act forming an intermediate enzyme-substrate complex. This complex is then converted to a reaction product and a liberated enzyme which continues its specific enzymatic function.

Enzymes provide several benefits when used for cleansing of the oral cavity. Proteases break down salivary proteins which are absorbed onto the tooth surface and form the pellicle; the first layer of resulting plaque. Proteases along with lipases destroy bacteria by lysing proteins and lipids which form the structural component of bacterial cell walls and membranes. Dextranases break down the organic skeletal structure produced by bacteria that forms a matrix for bacterial adhesion. Proteases and amylases, not only present plaque formation, but also prevent the development of calculus by breaking-up the carbohydrate protein complex that binds calcium, preventing mineralisation.

Enzymes useful in the present invention include any of the commercially available proteases, glucanohydrolases, endoglycosidases; amylases, nutanases, lipases and mucinases or compatible mixtures thereof. Preferred are the proteases, dextranases, endoglycosidases and multanases, most preferred being papain, endoglycidase or a mixture of dextranase and mutanase.

Other materials that can be used with the present invention include commonly known mouth and throat products. These products include, but are not limited to anti-fungal, antibiotic and analgesic agents.

Antioxidants are generally recognised as useful in compositions such as those of the present invention. Antioxidants that may be included in the oral care composition or substance of the present invention include, but are not limited to Vitamin E, ascorbic acid, Uric acid, carotenoids, Vitamin A, flavonoids and polyphenols, herbal antioxidants, melatonin, aminoindoles, lipoic acids and mixtures thereof.

Histamine-2(H-2)receptor antagonist compounds (H-2 antagonists) may be used in the oral care composition of the present invention. As used herein, selective H-2 antagonists are compounds that block H-2 receptors, but do not have meaningful activity in blocking histamine-1(H-1) receptors.

The oral healthcare substance of the present invention can be in a dry form e.g. solid particles, or in a fluid form e.g. in the form of a viscous liquid, paste, gel, solution or any other suitable form. Preferably, the substance is in the form of a gel, which may be an aqueous or non-aqueous (e.g. based on glycerol) gel, and may also include a gelling or thickening agent. It may be necessary to add additional gelling agents in the formula to help the active ingredients adhere to the tissues of the oral cavity. Suitable agents include both polymers with limited water solubility as well as polymers lacking water solubility. Suitable gelling agents useful in the present invention include carboxy-polymethylene; carboxymethyl cellulose, carboxypropyl cellulose, polyaxamers, carrageenan, Veegum, carboxyvinyl polymers, and natural gums such as gum karaya, xanthan gum, guar gum, gum arabic, gum tragacanth, and mixtures thereof. A preferable gelling agent for use in the present invention is carboxypolymethylene, obtained from B. F. Goodrich Company under tradename Carbopol®. Particularly preferable Carbopols include Carbopol 934, 940, 941, 956 and mixtures thereof.

If the oral care substance is an aqueous gel, the water present in the gel compositions should preferably be deionised and free of organic impurities. A pH adjusting agent may also be added to optimise the storage stability of the gel and to make the substance safe for the oral tissues. These pH adjusting agents, or buffers, can be any material which is suitable to adjust the pH of the oral care substance. Suitable materials include sodium bicarbonate, sodium phosphate, sodium hydroxide, ammonium hydroxide, sodium stannate, triethanolamine, citric acid, hydrochloric acid, sodium citrate, and combinations thereof. The pH adjusting agents are added in sufficient amounts so as to adjust the pH of the substance or composition to a suitable value, e.g. about 4.5 to about 11 (The material Proxigel has pH ca. 4.7 - 5.2), preferably from about 5.5 to about 8.5, and more preferably from about 6 to about 7. pH adjusting agents are generally present in an amount of from about 0.01 % to about 15 % and preferably from about 0.05 % to about 5%, by weight of the oral care substance.

An additional carrier material may also be added to the oral care substance or composition. These materials are generally humectants and include glycerin, sorbitol, polyethylene glycol and the like. The oral healthcare substance may comprise the substance itself, together with one or more substance enhancers, for example catalysts and/or potentiators to modify the release and/or activity of the substance. The device of the invention may additionally comprise additional substances such as flavours, colours etc, which may for example be deposited onto the surface of the strip or impregnated into the bulk of the strip in a manner analogous to the above-described oral healthcare substance and adhesive, *mutatis mutandis.*

The oral healthcare substance is preferably a tooth whitening substance, preferably a peroxide-containing gel. Suitable gels may be based on glycerol containing a peroxide such as hydrogen peroxide or an organic peroxide. A suitable gel is that disclosed in US-A-3,657,413, for example that sold under the trade mark PROXIGEL™ by The Block Drug Company (USA) (since acquired by GlaxoSmithKline plc). Other suitable peroxide-containing gels are for example disclosed in the art references cited above. The effectiveness of peroxide materials may be enhanced by means of a catalyst, i.e. a two-component peroxide-catalyst system. The strip may have the oral healthcare substance deposited upon its surface. For example a gel may be deposited directly as a layer on a surface of a plastically deformable strip, e.g. a wax strip, as described above. Alternatively a gel may be absorbed into the above-mentioned carrier layer, or impregnated into the bulk of the strip material, or deposited between layers of a multiple layered strip e.g. as described above.

Methods of depositing substances upon the surfaces of strip materials as described above are known, for example printing, e.g. silk screen printing, passing between impregnated rollers, dosing, a pump and nozzle, spraying, dipping etc. Methods of impregnating substances into the bulk of a strip material are also known, for example admixing the substance into the strip material and then forming the strip, or exposure of the strip to the substance under conditions which cause the substance to be impregnated into the strip. Alternatively example the strip material may be a foam material, particularly an open-cell foam material, and the substance may be impregnated into the strip material by introducing the substance into the cells of the foam. Some suitable technologies for deposition and impregnation of substances onto or into strip materials are known, for example from the transdermal patch art. The substance may subsequently be released from the strip by the action of moisture in the oral cavity dissolving the substance, or the effect of chemicals, enzymes etc, e.g. saliva amylases, present in the oral cavity.

The device of the invention may be marked with one or more visible symbol, e.g. text matter, a trade mark, a company logo, an area of colour, or an alignment feature such as a visible line or notch etc. to assist the user in applying the device to the teeth in a proper alignment. Such an alignment feature may for example comprise a symbol to show the user which way up the device should be whilst applying the device to the teeth, or which of a pair of the devices is intended for the upper teeth and which for the lower teeth. In this way the device may be made more visually attractive and/or easier to use. Such symbol(s) may be applied by conventional printing processes, e.g. silk screen printing, inkjet printing etc. to the surface of the plastically deformable material opposite to the surface on which is attached the layer of an absorbent material.

If such a visible symbol is applied to this surface a cover layer may be applied over the symbol, for example to protect it. This cover layer may be transparent or translucent to allow visible symbols to be seen through this layer.

Such a cover layer may be applied to the plastically deformable material by pressing, e.g. rolling, the material of the cover layer in contact with the plastically deformable material.

It may be preferable to provide an impermeable backing layer opposite the surface in contact with the tooth surface.

The invention may be realised in various forms.

The present invention comprises a strip of a plastically deformable material, to at least one surface of which is attached a layer of an absorbent material, with an oral healthcare substance on the layer of absorbent material.

The plastically deformable material may comprise the above-mentioned dental wax, and may suitably be 0.2-1.0mm thick, preferably 0.2-0.5mm thick, most preferably ca. 0.4 - 0.5 mm thick. Known types of dental wax can be rolled to such a thickness and formed into a strip shape.

The absorbent material in this preferred form is preferably a fabric, woven or non-woven, preferably non-woven. A suitable non-woven fabric is a polypropylene-viscose blend. A suitable thickness for the layer of absorbent fabric is 0.05 - 0.2mm, preferably 0.075 - 0.125mm, especially ca. 0.1mm. Suitably the total thickness of such a wax plus adsorbent material strip may be ca. 0.7 mm. The layer of absorbent fabric may be attached to the strip of plastically deformable material by known methods, for example by bringing the fabric into contact with the surface of the strip and applying pressure, e.g. in a calendering process. In this way the plastically deformable material may be forced among the fibres of the absorbent fabric to thereby generate a bond.

In this preferred form the oral healthcare substance is preferably the above-mentioned peroxide-containing gel. Typically the gel may be applied to the absorbent fabric using conventional methods for example applying by means of a roller. Such a gel can soak into the adsorbent material and can be completely absorbed by the material, or some may remain unabsorbed as a surface layer. It is found that the absorbent fabric helps to retain the substance on the device, because a substance such as the above-mentioned tooth whitening gel can be absorbed into the absorbent fabric. The above-mentioned preferred thickness of fabric is found suitable to retain a sufficient quantity of such a gel on the device. The absorbent fabric also helps to prevent the substance, if it is a fluid gel, from being squeezed out from between the strip and the teeth surfaces when the device is pressed against the teeth. Typically a loading of 250-750mg of such a peroxide-containing gel substance may be used, preferably 250-500mg. It should be noted that not all of the oral healthcare substance loaded onto the device might actually ultimately contact the oral surfaces, and in a test using a peroxide-containing gel ca. 70% of substance loaded was actually transferred to tooth surfaces.

In this preferred form the strip of plastically deformable material, e.g. the wax, may be marked with one or more visible symbol as mentioned above, typically applied to the surface of the plastically deformable material opposite to the surface on which is attached the layer of an absorbent material. When such a visible symbol is applied it is preferred to apply a cover layer as mentioned above over the symbol. This cover layer preferably comprises the same material, e.g. the wax, as the layer of plastically deformable material so that the symbol is in effect embedded in the plastically deformable material, or sandwiched between the strip and the cover layer of wax material.

Such a cover layer may be applied to the plastically deformable material by pressing, e.g. rolling, the material of the cover layer in contact with the plastically deformable material.

A typical process for making the above-mentioned preferred form of the device of the invention using a dental wax as provided in the form of a sheet or reel as the plastically deformable material, may involve the following steps.

If necessary, cleaning the surface of the wax material. As provided dental wax may have a surface contamination of for example coconut oil, and typically wiping the surface with for example a non-woven cloth may be suitable to clean the surface. A continuous extrusion process may be used to prepare wax substantially free of surface contamination.

A visible symbol may be printed upon a first surface of a first sheet of the wax material. Suitably one or more inkjet printer may be used for this, using a suitable ink, preferably an ink which is orally acceptable.

A second, cover sheet of the wax material may be laminated to the first surface of the first sheet, to sandwich the printed symbol between the first and second sheets, and in effect to embed the printed symbol in the laminated sheets of dental wax. This may be done by pressing, e.g. rolling the first and second sheets together between rollers at a suitable pressure, and elevated temperature if found necessary (e.g. rollers at a surface temperature ca. 85° C, with a speed of 200 mm/sec), which can easily be determined by those skilled in the art.

The laminated sheets may then be sized by compressing them, e.g. between rollers, to compress them to a suitable thickness, e.g. as mentioned above.

The layer of absorbent fabric may then be attached to the laminated wax strips by known methods, for example by bringing the fabric into contact with a surface of the first sheet opposite to the first surface, and applying pressure. For example the laminated sheets of wax and the fabric may be rolled together between rollers. In this way the plastically deformable material may be forced among the fibres of the absorbent fabric to thereby generate a bond.

In the above-mentioned steps where the wax sheets are compressed between rollers it may be necessary to provide a sheet of a "sacrificial" material e.g. a paper between the wax sheet and a roller to prevent the wax sticking to the roller. For example a preferred way of providing the wax may be to continually cast it as a sheet on a release paper carrier.

The sheet of laminated material may then be cut to shape, for example using a die cutter. The oral healthcare substance may then be applied to the layer of absorbent material, using a conventional method such as applying by means of a roller, or preferably using a delivery pump and nozzle.

In another embodiment the oral healthcare substance may be encapsulated. Encapsulation may for example be in micro-capsules, or macro-capsules. Methods of micro-encapsulation are known, for example in which a droplet of a substance is enclosed in a liquid phase within a layer of an encapsulation material, and then separated from the liquid. Such capsules may be deposited on or adjacent the surface of the sheet, and may for example be burst physically or chemically, e.g. by pressure e.g. as the strip is applied to the tooth surface or by subsequent bite action, by breaching of the capsule wall under the action of the temperature, moisture, pH, chemicals or enzymes in the mouth environment etc. For example capsules of oral healthcare substance may be attached to the surface of the strip, e.g. by means of adhesive or by embedding the capsules in the strip material. For example a thin layer of adhesive may be deposited on the surface of the strip, and capsules of the oral healthcare substance may be embedded at least partly if not completely within this adhesive layer, or may sit upon the surface of this adhesive layer.

In another embodiment the oral healthcare substance may be provided in granules, e.g. pellets or micropellets, which may release their content under the influence of the mouth environment, for example moisture, chemicals or enzymes in the mouth, and may be coated to achieve this release. Methods of granulation and pelletizing are known, as are coating polymers such as the known Eudragit™ polymers which dissolve at specified pH. Such granules may be deposited on or adjacent the surface of the strip. For example granules of oral healthcare substance may be attached to the surface of the strip, e.g. by means of adhesive or by embedding the granules in the strip material. For example a thin layer of adhesive may be deposited on the surface of the strip, and granules of the oral healthcare substance may be embedded at least partly if not completely within this adhesive layer.

For example capsules and/or granules of oral healthcare substance may be located substantially uniformly over the strip surface.

In embodiments in which the oral healthcare substance is provided in the form of capsules or granules deposited on a surface of the strip, e.g. as described above, the capsules or granules may be covered by a porous membrane layer, e.g. of a non-woven fabric material as described above. Such a membrane layer may help to retain the capsules or granules on the surface of the strip, and in the case of capsules may also help to retain capsule casing debris when the capsules have opened to release their content, whilst allowing active material content to pass through. Such an embodiment may for example be made by depositing the granules and/or capsules on the surface of the strip, and then laminating the membrane layer onto the strip over the capsules. For example capsules and/or granules may be deposited in a region on the strip, and the membrane layer may be bonded to the strip around the region.

Potentiators and/or catalysts as mentioned above may for example be provided in other capsules or granules which release their contents in the mouth. Alternatively such potentiators and/or catalysts may be provided externally to such capsules or granules of substance, e.g. as an outer coating on the capsules or granules, or deposited on or impregnated in the strip material adjacent to the capsules or granules, e.g. in a layer of material to which the capsules or granules are attached or in which they are embedded.

In another embodiment the flexible strip strip material may be inherently adherent to a tooth surface. For example the strip material may adhere to the tooth surface by surface tension under the influence of moisture in the mouth. For example the strip material may be a hydrophilic polymer having for example muco-adherent properties.

Mechanical adhesion between the strip and tooth or other oral surface is provided by the strip comprising a plastically deformable material, particularly the above-mentioned wax, which can be plastically deformed by the user to conform the strip to the contours of the tooth or other oral surface, particularly fitting into convavities and spaces between teeth, and so adhere thereto by mechanical gripping. A layer of an oral healthcare substance such as the above-described gel materials may be deposited thereon. Such gripping may be enhanced by e.g. surface effects between the strip and the surface such as formation of a partial vacuum or surface tension effects. For example the strip may have anchors on its surface, positioned at approximately the spacings of gaps between teeth, and these anchors may fit into the gaps between the teeth.

The strip may for example be stretchable, for example softening under the action of moisture, chemicals or enzymes in the mouth so that it can be adjusted to the spacings of gaps between an individual user's teeth. Another form of "mechanical" adhesion may be provided by a strip material which shrinks in contact with the tooth surface, for example in contact with moisture, chemicals or enzymes in the mouth, or any other feature of the mouth environment, so that the shrunken strip can physically grip the surface of the tooth.

Suitable shapes of the device of the invention, for example the above-mentioned preferred form of the device, will now be discussed. The device of the invention is suitably of elongate shape, of a length sufficient that when placed against the front surface of the teeth of a user it extends across a plurality of teeth. Suitably the device is sufficiently long to extend at least die user's canine teeth, for example to the pre-molar teeth.

The device is suitably of sufficient width that when placed against the user's teeth it extends from the gumline at least to the crowns of the front teeth distant from the gumline. Suitably the width is such that in an unfolded state the strip has an unfolded width greater than the height of the teeth from the gumline to the crown, and at least part of the strip may be folded about a substantially longitudinal fold axis so as to bend or fold over the crowns and contact the crowns and rear surfaces of the user's teeth, i.e. so that in cross section the folded part of the device is substantially of a "U" or "V" shape, with two limbs linked at a fold axis each limb with an inward surface facing into the "bite" of the "U" or "V", with the oral healthcare substance present on this inward, tooth-contacting surface. Such a strip can be applied to a tooth so that the inward facing surface of a first limb can be applied to one surface of a tooth, e.g. a surface that faces into the interior of the mouth, and the inward facing surface of a second limb can be applied to an opposite surface of the tooth, e.g. a surface that faces outwardly, e.g. a front surface of the tooth.

In the above mentioned preferred form of the device it is believed that the physical, i.e. mechanical conformation of the plastically deformable wax to the shape of the teeth, e.g. fitting into the interdental spaces etc., causes sufficient attachment of the preferred form of the device to the user's teeth. Pressure may be applied by the user to achieve this. When the preferred peroxide-containing gel is used as the oral healthcare substance with the preferred form of the device then this gel may in fact act as a slimy lubricant reducing adhesion between the device and a tooth surface, rather than as an adhesive.

The device of the invention may be of various shapes, depending upon whether it is to be applied to the upper or lower teeth. In general, for both upper and lower teeth the device may be substantially rectangular or trapezoidal, for example with rounded corners and/or ends.

Suitably to be applied to the upper teeth the device may be substantially rectangular with concavely curved long sides. To be applied to the lower teeth the device may be substantially rectangular with convexly bowed long sides, or of a generally rectangular shape but with a concave curved long side or a concave indentation in a long side.

Suitably, in particular for application to the upper teeth the device may have a tab extending from a long side. For example such a tab may be substantially rectangular. Such a tab may be used to assist the user in manipulating the device and applying it to the tooth surfaces.

The dimensions of the device will in practice be determined principally by the typical dimensions of a user's teeth. Typically the device may be ca. 4 - 10cm long x ca.1.0 - 3cm wide, typically ca. 7-8 cm long x ca.2 - 2.5 cm wide . This is found to be a suitable width to enable the strip to be applied to both the front and rear surfaces of the user's teeth, by folding the strip into a "U" shape over the crowns of the teeth. These sizes are suitable for a loading of a peroxide-containing gel as mentioned above of ca. 200 - 750 mg.

If the substance is impregnated into the strip material so as to be released from the strip material onto the tooth surface, variants of the above-described options may be used.

Typical use of the device of this invention will now be described. The device of this invention may be applied manually to a user's tooth surface(s), for example using the user's fingers or using an applicator device to position the strip on or adjacent to the tooth surface. The device is applied by the user to his/her teeth, with the length dimension of the strip aligned with the line of the user's teeth, the substance-bearing surface in contact with the front surface of the teeth, and the device is pressed against the front surface of the teeth.

When using the preferred form of the device, the plastic deformation of the strip material causes the device to become shaped to the profile of the teeth, and to fit into the gaps between the teeth. If the device has a width greater than the length of the teeth it may be folded over the distant ends of the teeth to contact the rear surfaces of the teeth and be pressed against the rear surface, thereby becoming shaped to the rear surfaces of the teeth. A tab, if present, can assist the user in manipulating the device into place.

The plastic deformation of the preferred form of the device causes the device to remain in its deformed shape after the deforming pressure has ceased, until the device is removed again, e.g. by the user. The adapting of the device to fit the shape of the teeth, combined with any folding of the device over the distant ends to contact the rear surfaces of the teeth, is the principal force holding the device in place against the teeth. The device may also be held against the teeth by one or more other forces in addition to the grip provided by this plastic deformation, for example surface tension from mouth fluids such as saliva, the pressure of adjacent mouth surfaces such as the lips against the device.

The device is left in contact with the user's teeth for a sufficient length of time for the oral healthcare substance to have its effect. The period of use will depend upon the particular user, convenience, the state of the user's teeth, the healthcare effect desired, e.g. degree of whitening required etc. This period may for example be as short as 10 minutes, or may be longer for example 2 hours, and the device may be applied for plural sessions each day, e.g. two 30 minute sessions per day. The use of the device may be repeated for a similar length of time over a course of several days, e.g. 7 to 14 days until a desired extent of the oral healthcare effect, e.g. tooth whitening, has been achieved. Whilst the device is in contact with the teeth, the oral healthcare substance is held in immediate contact with the teeth surfaces and is protected from removal as a result of washing with mouth fluids or contact by adjacent mouth surfaces by the covering layer of the plastically deformable material. After use the device may simply be removed from the teeth and disposed of, and the user may rinse his/her mouth, and/or brush his/her teeth, to remove residual healthcare substance if desired. By use of the gel materials described which have little or no adhesive effect but use the mechanical adhesion achieved by mechanical gripping of the teeth by for example a plastically deformable wax material, it is particularly easy to rinse the substance off the teeth and oral tissues after use.

The device may be supplied for use with a protective cover film over the oral healthcare substance to protect it from contamination and/or loss. This film may be an easy to peel off film.

Individual devices of the invention may be supplied contained for example in sachets, e.g. correspondingly sized generally flat envelopes, and suitably such sachets can be opened at one face thereof so that the device contained therein can be removed without any longitudinal sliding motion which might scrape off the substance. Suitably a sufficient number of such device-conmmmg sachets may be contained in an outer pack to enable a user to complete a course of use. Such a pack, or individual sachets, may be marked with instructions for use, or the pack may contain an instruction leaflet.The invention will now be described by way of example only with reference to the accompanying figures which show:
Fig. 1 shows a cross section through a preferred form of the device of this invention
Fig. 2 shows a cross section through another preferred form of this invention.
Figs. 3-5 show plan views of a preferred form of the device of this invention suitable for the upper teeth
Figs. 6-8 show plan views of a preferred form of the device of this invention suitable for the lower teeth
Figs. 9 and 10 show the device of Figs. 1-6 in place on the user's teeth
Fig. 11 shows a section through a tooth with the device of Figs. 17-23 in place.
Figs. 12 and 13 show a suitable package for the device of the invention.

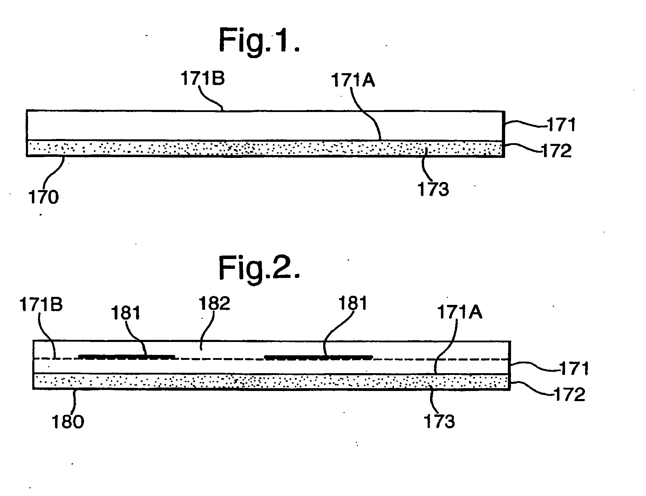

Figs. 1 to 13 relate to a preferred form of the device of this invention.

Referring to Fig. 1 a device 170 of the invention is shown in section. The device comprises a strip 171 of a plastically deformable material, being a dental wax (SW12 or SW13 sheet wax, obtainable from The Kindt-Collins Co., Cleveland, Ohio, USA), or waxes S & P wax 1, wax X1 or dental wax II from Strahl & Pitsch (favoured for its low odour) or a wax from Witco known as 13324. These waxes are available in sheets and can be used in this form or cast into a sheet with a thickness ca. 0.3mm. Bonded to one surface 171A of the strip 171 is a layer 172 of an hydrophobic absorbent fabric, being a non-woven polypropylene / viscose blend, (grade HYN-35 non woven obtainable from BFF Non Wovens, Somerset, GB). The layer 172 is ca. 0.1mm thick. Opposite to surface 171A is a surface 171B, termed the "upper surface" of the strip 171. The layer 172 is ca. 0.5 mm thick in its uncompressed form and is bonded to strip 171 by starting with a strip 171 ca. 0.5mm thick, bringing it into contact with the fabric, and squeezing the strip 171 and the fabric between heated rollers (e.g. rollers at a surface temperature ca. 85° C, with a speed of 200 mm/sec). The material of the strip 171 is thereby forced by pressure among the fibres of the fabric to bond them together. Deposited on the layer 172, and absorbed thereby, is a tooth-whitening gel 173 being the tooth whitening gel substance PROXIGEL™ sold by The Block Drug Company. The gels disclosed as Examples 1 or 2 of US 3,657,413 are also suitable. The gel 173 is applied to the fabric 172 by for example using a roller or a brush, and is mostly absorbed into the fabric layer but some may lie upon the surface of the layer 172.

Referring to Fig. 2 another form 180 of the invention is shown in a cross section similar to Fig. 1, and in which corresponding features are numbered correspondingly. Visible symbols 181 have been printed onto the surface 171B using a conventional printing technique, for example ink jet printing. The above-mentioned wax is relatively easy to print on. On the upper surface 171B of the plastically deformable material 171, there is a cover layer 182, being the same wax material as the plastically deformable material 171, so that the symbols 181 are sandwiched between layers 171 and 182, in effect the symbols 181 being embedded in the plastically deformable material 171, 182. This cover layer 182 is translucent, and allows the visible symbols 181 to be seen through this layer 182. The cover layer 182 is applied to layer 171 by for example casting (e.g. slot die casting) the second wax layer 182 directly onto the first layer 171. Alternately the second layer 182 may be applied by pressing the strip 171 and a sheet of the material of layer 182 together, with heating, so that the strip 171 and layer 182 merge into a monolithic mass of the wax. The merged layers 171 and 182 can then be sized e.g. by pressing to a suitable thickness e.g. ca. 0.5 mm.

The layer of absorbent material 172 may then be applied to the layer 171 in a manner analogous to that described above for Fig. 1. The overall thickness of the three layers 182, 171, 172 is ca. 0.7mm.

The pressing operations described above may be accomplished by passing the various strips through compressing rollers, in a manner conventional to the those skilled in the art.

In the devices specifically illustrated in Figs. 1 and 2 the typical loading of the gel 173 is ca. 250-350 mg per strip.

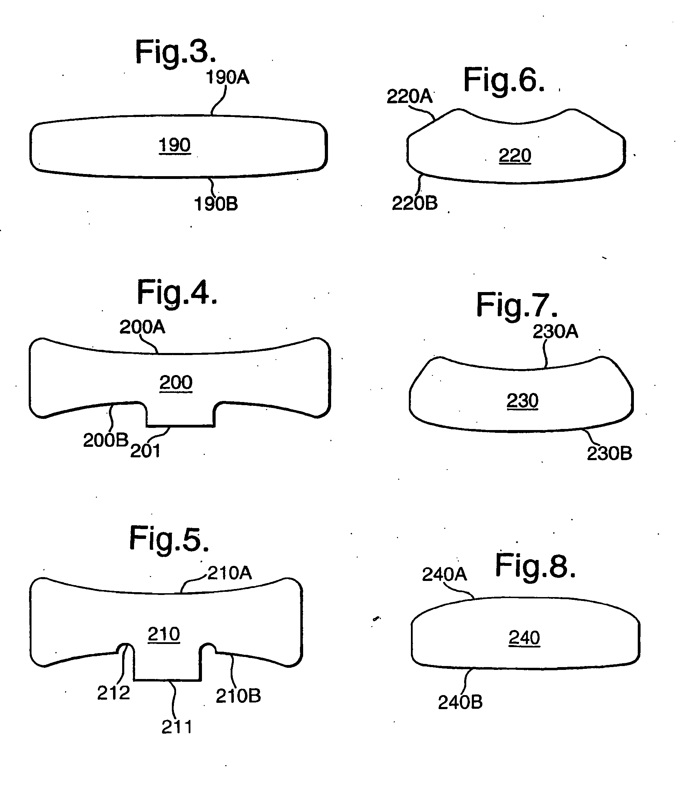

Referring to Figs. 3-8 various overall shapes of the preferred form of the device of the invention are shown.

Figs. 3, 4 and 5 show devices suitable for the upper teeth. The device 190 of Fig. 3 is generally rectangular, ca. 7.5 cm x 1.75 cm, and has slightly convex curved long sides 190A and 190B, with rounded corners. The devices 200 and 210 of Figs. 4 and 5 are generally rectangular, 200 being ca. 7.5cm x 1.75cm, and 210 being ca. 6 x 2.3 cm, and have slightly concave curved long sides 200A, 200B, 210A, 210B. From the long side 200B, 210B of each of device 200, 210 projects a generally rectangular tab 201, 211. In the angles between the edges of the tab 211 and the long side 210B of device 210 are small notches 212.

Figs. 6, 7, and 8 show devices suitable for the lower teeth. The devices 220, 230, 240 are all generally rectangular being ca. 7 cm x 2 cm. The devices 220, 230 have a slightly convex curved lower side 220A, 230A, but a slightly concave curved upper edge 220B, 230B. The device 240 of Fig. 8 has a straight lower side 240A and a slightly concave upper side 240B.

A combination of the device 210 for the upper teeth and 230 for the lower teeth is preferred.

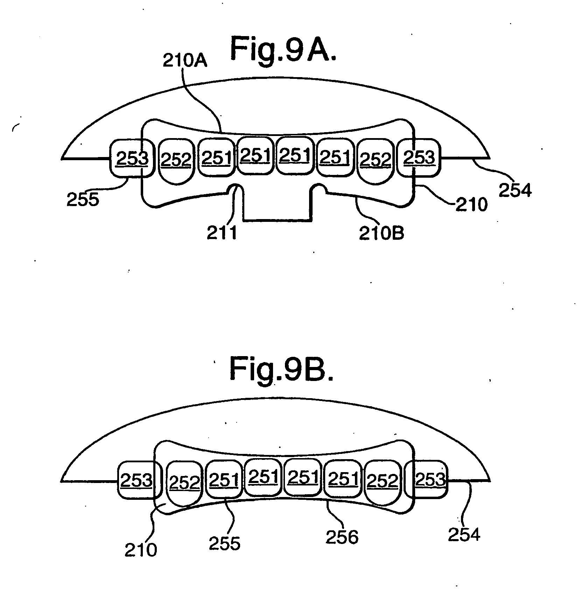

Figs. 9, 10 and 11 show a device 210 as shown in Fig. 5 applied to the teeth.

Referring to Fig. 9, a device 210 as shown in Fig. 5 has been applied to the user's upper teeth in a manner as follows. Fig. 9A shows the front row of the user's upper teeth, being incisor teeth 251, canine teeth 252 and the first pre-molar teeth 253. The view in Fig 9 is looking straight at the teeth from in front of the user. The device 210 has been applied to the front surface of the teeth 251-253, with its upper long side 210A overlapping the gumline 254. The opposite long side 210B extends beyond the crowns 255 (generally) of the teeth 251-253 distant from the gumline 254, with the tab 211 extending downwardly. As shown in Fig. 9B The device 210 has been folded or bent along a longitudinal fold line 256 so as to come into contact with the rear surfaces of the teeth 251-253. The length of the device 210 is such as to cover the ends of the canine teeth 252 of the user. The absorbent fabric 172, with its loading of the tooth whitening substance 173, is in contact with the surfaces of the teeth 251 - 233. A device 230 may be applied analogously to the front surface of the lower teeth, again comprising incisor teeth, canine teeth and pre-molar teeth. Analogously the device 230 also folds or bends at its fold line to come into contact with both the crowns of the teeth and the rear surfaces of the teeth.

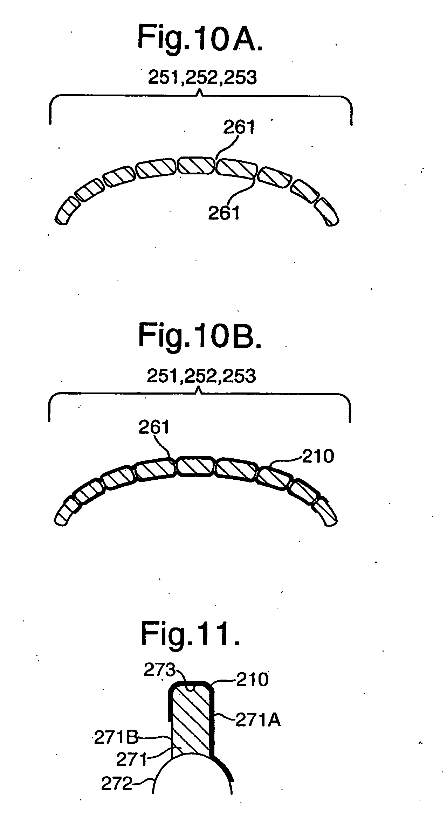

Referring to Fig. 10, a plan view of the line of the user's teeth 251-253 is shown in Fig. 10A, extending from the incisor teeth 251 to the first pre-molars 253. As shown in Fig 10B the device 210 has been applied to the front surfaces of the teeth 251-253 and a firm pinching action has been applied to plastically deform the layer of wax 171. The absorbent fabric 172, with its loading of the tooth whitening substance 173, is in contact with the surfaces of the teeth 251 - 233. The pinching pressure has caused the device 210 to accommodate itself to the overall curve of the front surface of the teeth 251-253, and also to the concavities of the gaps 261 between the user's teeth 251 - 253. The device 260 has also been folded over the crowns of the teeth 251-253, so that the device 260 has also accommodated itself to the overall curve of the back surface of the teeth 251-253, and to the concavities of the gaps 261 between the user's teeth at the back surface.

Fig. 11 shows a cross section through an individual tooth 271 of the row 251-253 shown in Fig. 26 projecting from the gum 272, with the device 210 applied to its surface, with the fabric layer 172 and substance 173 in contact with the tooth 271. A firm pinching action applied by the user has caused the strip 210 to accommodate itself to the curves of the front teeth surface 271A and also to fold over the crown 273 of the tooth 271, and to contact the rear surface of the tooth 271B, accommodating itself to the curves of the rear surface 271B also.

This close fitting of the device 210 to the overall and individual shape of the teeth 251-253 achieved by the plastic deformation of the wax layer 171, 182 causes the device 210 to grip onto the user's teeth by friction and deadfold and to be retained in place during a suitable period of use. The device 210 may be retained in contact with the teeth 251-253 for a suitable period before being removed by the user, involving lifting the device 210 off the teeth, followed by rinsing the mouth if felt necessary.

Devices 170-310 may be applied simultaneously to both the user's upper and lower teeth. It has been found that the wax strips 171 of the respective upper and lower strips do not generally stick together on contact whilst both upper and lower strips are worn. However only one device 170-240 may be used at a time, applied to only the upper or lower teeth.

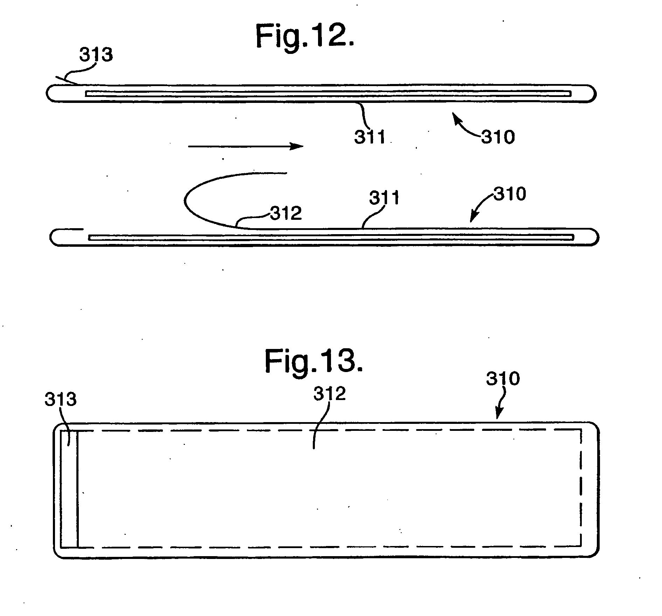

Figs. 12 and 13 show a sachet 310 suitable for a device 170, 180, 190, 200, 210, 220, 230, 240, of this invention. The sachet 310 comprises a flat envelope defined by an envelope wall 311 of a foil laminate of a shape closely conforming to the shape of the device e.g. 170. A section 312 of the envelope wall 311 of the sachet 310 can be peeled away by pulling a tab 313, to form an opening in the sachet 310 by which the device e.g. 170 contained therein may be removed for use.

## Claims

1. A delivery device (170) for delivering an oral healthcare substance to the oral surfaces of teeth, gingival and/or mucosal tissues, comprising;
a strip (170) of an orally acceptable flexible material, having a strip surface capable of being applied to a tooth surface and/or adjoining soft tissue,
the strip having an oral healthcare substance deposited upon a strip surface thereof, and/or impregnated into its bulk, the substance being capable of transference from the strip surface to the tooth surface and/or adjoining soft tissue,
the strip (170) comprising a layer (171) of a plastically deformable material which can be deformed by the user using finger or hand pressure to fit the device to the overall shape of the user's teeth to thereby grip the teeth,
**characterised in that** the strip (170) further comprises a layer of an absorbent material which is attached to at least one surface of said plastically deformable layer (171),
the oral healthcare substance being on the layer (172) of absorbent material.

2. A device (170) according to claim 1 ***characterised* in that** the plastically deformable material (171) comprises a wax.

3. A device (170) according to claim 2 ***characterised* in that** the wax comprises a dental wax.

4. A device (170) according to any one of claims 1 to 3 ***characterised* in that** the plastically deformable material is capable of being plastically deformed so that the device can be folded or bent over the user's teeth from the front to the back surface of the teeth, so as to be in contact with both the front and back surfaces.

5. A device (170) according to any one of the preceding claims ***characterised* in that** the absorbent material (172) is an absorbent fabric.

6. A device (170) according to claim 5 ***characterised* in that** the fabric (172) is non-woven.

7. A device (170) according to claim 6 ***characterised* in that** the non-woven fabric is a polypropylene-viscose blend.

8. A device (170) according to any one of claims 5, 6 or 7 ***characterised* in that** the absorbent fabric (172) is bonded to the plastically deformable material by the plastically deformable material (171) having been forced among the fibres of the fabric (172) to provide a bond

9. A delivery device (180) according to any one of claims 1 to 8 ***characterised* in that** the device (180) is marked with one or more visible symbol (181), and a cover layer (182) is applied over the one or more symbol (181).

10. A device (180) according to claim 9 ***characterised* in that** the cover layer (182) is the same material as the plastically deformable material so that the symbol (181) is embedded in the plastically deformable material.

11. A delivery device (170,180) according to any one of the preceding claims ***characterised* in that** the oral care substance is a tooth whitening substance.

12. A delivery device (170,180) according to any one of the preceding claims ***characterised* in that** the oral care substance is in the form of a gel.

13. A delivery device (170,180) according to claim 12 ***characterised* in that** the oral healthcare substance is a peroxide-containing gel.

14. A device (170,180) according to any one of the preceding claims ***characterised* by** an elongate shape of a length sufficient that when placed against the front surface of the teeth of a user it extends across a plurality of teeth, and of sufficient width that when placed against the user's teeth it extends from the gumline at least to the crowns of the front teeth distant from the gumline.

15. A device (170,180) according to claim 14 ***characterised* in that** the width is such that in an unfolded state the strip has an unfolded width greater than the height of the teeth from the gumline to the crown, and at least, part of the strip may be folded about a substantially longitudinal fold axis so as to bend or fold over the crowns and contact the crowns and rear surfaces of the user's teeth.

16. A device (200,210) according to claim 14 or 15 ***characterised* by** being substantially rectangular with concavely curved long sides.

17. A device (190,220, 230,240) according to claim 14 or 15 ***characterised* by** being substantially rectangular with convexly bowed long sides, or of a generally rectangular shape but with a concave, curved long side or a concave indentation in a long side.

18. A device according to claim 16 or 17 ***characterised* by** a tab (201,211) extending from a long side.

19. A process for making a device (170,180) according to any one of claims 1 to 18 ***characterised* by** bringing the absorbent material (172) into contact with the surface of the strip (171) and applying pressure, then applying the oral healthcare substance to the absorbent material (172).

20. A process according to claim 19 ***characterised* by** providing a dental wax in the form of a sheet (171), printing a visible symbol (181) upon a first surface (171B) of the sheet (171) of the wax material, laminating a second, cover sheet (182) of the wax material to the first surface of the sheet, to sandwich the printed symbol between the first and second sheets, sizing the laminated sheets by compressing them to a suitable thickness, attaching the layer of absorbent fabric (172) to the laminated wax strip, cutting the sheet of laminated material to shape, applying the oral healthcare substance to the layer of absorbent material.

## Patentansprüche

1. Abgabevorrichtung (170) zum Abgeben einer Mundgesundheitspflege-Substanz an die Mundoberflächen von Zähnen, Zahnfleisch- und/oder Schleimhautgewebe, mit:
einem Streifen (170) aus einem oral akzeptablen flexiblen Material, mit einer Streifenoberfläche, die imstande ist, auf eine Zahnoberfläche und/oder angrenzendes Weichgewebe aufgebracht zu werden,
wobei der Streifen eine Mundgesundheitspflege-Substanz auf einer Streifenoberfläche von ihm abgelegt, und/oder in seine Hauptmasse imprägniert aufweist, wobei die Substanz zur Übertragung von der Streifenoberfläche an die Zahnoberfläche und/oder angrenzendes Weichgewebe imstande ist,
wobei der Streifen (170) eine Lage (171) aus einem plastisch verformbaren Material umfasst, die durch den Nutzer verformt werden kann, der einen Finger- oder Handdruck verwendet, um die Vorrichtung der Gesamtform der Zähne des Nutzers anzupassen, um **dadurch** die Zähne zu greifen,
**dadurch gekennzeichnet, dass** der Streifen (170) ferner eine Lage aus einem absorbierenden Material umfasst, die an zumindest einer Oberfläche der plastisch verformbaren Lage (171) angebracht ist,
wobei die Mundgesundheitspflege-Substanz auf der Lage (172) aus absorbierendem Material ist.

2. Vorrichtung (170) nach Anspruch 1, **dadurch gekennzeichnet, dass** das plastisch verformbare Material (171) ein Wachs umfasst.

3. Vorrichtung (170) nach Anspruch 2, **dadurch gekennzeichnet, dass** das Wachs ein Dentalwachs umfasst.

4. Vorrichtung (170) nach einem der Ansprüche 1 bis 3, **dadurch gekennzeichnet, dass** das plastisch verformbare Material imstande ist, so plastisch verformt zu werden, dass die Vorrichtung über die Zähne des Nutzers von der vorderen zu der hinteren Oberfläche der Zähne gefaltet oder gebogen werden kann, um sowohl mit der vorderen als auch der hinteren Oberfläche in Kontakt zu sein.

5. Vorrichtung (170) nach einem der vorhergehenden Ansprüche, **dadurch gekennzeichnet, dass** das absorbierende Material (172) ein absorbierender Stoff ist.

6. Vorrichtung (170) nach Anspruch 5, **dadurch gekennzeichnet, dass** der Stoff (172) ein Vlies ist.

7. Vorrichtung (170) nach Anspruch 6, **dadurch gekennzeichnet, dass** der Vliesstoff eine Polypropylen-Viskose-Mischung ist.

8. Vorrichtung (170) nach einem der Ansprüche 5, 6 oder 7, **dadurch gekennzeichnet, dass** der absorbierende Stoff (172) mit dem plastisch verformbaren Material **dadurch** verbunden ist, dass das plastisch verformbare Material (171) unter die Fasern des Stoffes (172) gedrängt wurde, um eine Verbindung vorzusehen.

9. Abgabevorrichtung (180) nach einem der Ansprüche 1 bis 8, **dadurch gekennzeichnet, dass** die Vorrichtung (180) mit einem oder mehreren sichtbaren Symbolen (181) markiert ist, und wobei eine Decklage (182) über das eine oder mehrere Symbol (181) aufgebracht ist.

10. Vorrichtung (180) nach Anspruch 9, **dadurch gekennzeichnet, dass** die Decklage (182) das gleiche Material wie das plastisch verformbare Material ist, so dass das Symbol (181) in das plastisch verformbare Material eingebettet ist.

11. Abgabevorrichtung (170, 180) nach einem der vorhergehenden Ansprüche, **dadurch gekennzeichnet, dass** die Mundpflege-Substanz eine Zahnweiß-Substanz ist.

12. Abgabevorrichtung (170, 180) nach einem der vorhergehenden Ansprüche, **dadurch gekennzeichnet, dass** die Mundpflege-Substanz in der Form eines Gels ist.

13. Abgabevorrichtung (170, 180) nach Anspruch 12, **dadurch gekennzeichnet, dass** die Mundgesundheitspflege-Substanz ein Peroxid-Gel ist.

14. Vorrichtung (170, 180) nach einem der vorhergehenden Ansprüche, **gekennzeichnet durch** eine längliche Form mit einer Länge, die ausreichend ist, dass, wenn sie gegen die vordere Oberfläche der Zähne eines Nutzers platziert ist, sie sich über eine Vielzahl von Zähnen erstreckt, und mit ausreichender Breite, dass, wenn sie gegen die Zähne des Nutzers platziert ist, sie sich von der Zahnfleischlinie zumindest zu den von der Zahnfleischlinie entfernten Scheitelpunkten der Vorderzähne erstreckt.

15. Vorrichtung (170, 180) nach Anspruch 14, **dadurch gekennzeichnet, dass** die Breite derart ist, dass der Streifen in einem ungefalteten Zustand eine ungefaltete Breite aufweist, die größer als die Höhe der Zähne von der Zahnfleischlinie zu dem Scheitelpunkt ist, und wobei zumindest ein Teil des Streifens um eine im Wesentlichen längliche Falzachse gefaltet werden kann, um über die Scheitelpunkte gebogen oder gefaltet zu sein und die Scheitelpunkte und hinteren Oberflächen der Zähne des Nutzers zu berühren.

16. Vorrichtung (200, 210) nach Anspruch 14 oder 15, **dadurch gekennzeichnet, dass** sie im Wesentlichen rechteckig mit konkav gebogenen langen Seiten ist.

17. Vorrichtung (190, 200, 230, 240) nach Anspruch 14 oder 15, **dadurch gekennzeichnet, dass** sie im Wesentlichen rechteckig mit konvex ausgebauchten langen Seiten ist, oder eine im Allgemeinen rechteckige Form aufweist, aber mit einer konkav gebogenen langen Seite oder einer konkaven Einkerbung in einer langen Seite.

18. Vorrichtung nach Anspruch 16 oder 17, **gekennzeichnet durch** eine Fahne (201, 211), die sich von einer langen Seite erstreckt.

19. Verfahren zur Herstellung einer Vorrichtung (170, 180) nach einem der Ansprüche 1 bis 18, **gekennzeichnet durch** in Kontakt Bringen des absorbierenden Materials (172) mit der Oberfläche des Streifens (171) und Aufbringen von Druck, wobei dann die Mundgesundheitspflege-Substanz auf das absorbierende Material (172) aufgebracht wird.

20. Verfahren nach Anspruch 19, **gekennzeichnet durch** Vorsehen eines Dentalwachses in der Form eines Bogens (171), Drucken eines sichtbaren Symbols (181) auf eine erste Oberfläche (171B) des Bogens (171) aus dem Wachsmaterial, Laminieren eines zweiten Deckbogens (182) aus dem Wachsmaterial auf die erste Oberfläche des Bogens, um das gedruckte Symbol zwischen dem ersten und dem zweiten Bogen sandwich-artig anzuordnen, Bestimmen der Größe der laminierten Bögen **durch** Zusammendrücken von ihnen auf eine geeignete Dicke, Anbringen der Lage aus absorbierendem Stoff (172) auf den laminierten Wachsstreifen, Schneiden des Bogens aus laminiertem Material in Form, Aufbringen der Mundgesundheitspflege-Substanz auf die Lage aus absorbierendem Material.

## Revendications

1. Dispositif de distribution (170) destiné à délivrer un produit de soin bucco-dentaire sur les surfaces buccales des dents, tissus gingivaux et/ou muqueux, comprenant :
une bande (170) réalisée à partir d'un matériau souple oralement acceptable, présentant une surface de bande pouvant être appliquée sur une surface dentaire et/ou les tissus mous adjacents,
la bande présentant un produit de soin bucco-dentaire déposé sur une surface de bande de cette dernière, et/ou imprégné dans son volume, le produit pouvant être transféré de la surface de bande à la surface dentaire et/ou aux tissus mous adjacents,
la bande (170) comprenant une couche (171) de matériau à déformation plastique qui peut être déformée par l'utilisateur au moyen d'une pression du doigt ou de la main, pour ajuster le dispositif à la forme globale des dents de l'utilisateur, et ainsi le faire adhérer aux dents,
**caractérisé en ce que** la bande (170) comprend en outre une couche d'un matériau absorbant qui est fixée à au moins une surface de ladite couche à déformation plastique (171), le produit de soin bucco-dentaire se trouvant sur la couche (172) du matériau absorbant.

2. Dispositif (170) selon la revendication 1, **caractérisé en ce que** le matériau à déformation plastique (171) comprend une cire.

3. Dispositif (170) selon la revendication 2, **caractérisé en ce que** la cire comprend une cire dentaire.

4. Dispositif (170) selon l'une quelconque des revendications 1 à 3, **caractérisé en ce que** le matériau à déformation plastique peut présenter une déformation plastique de telle sorte que le dispositif puisse être plié ou courbé sur les dents de l'utilisateur de la surface avant à la surface arrière des dents, de manière à être en contact à la fois avec les surfaces avant et arrière.

5. Dispositif (170) selon l'une quelconque des revendications précédentes, **caractérisé en ce que** le matériau absorbant (172) est un tissu absorbant.

6. Dispositif (170) selon la revendication 5, **caractérisé en ce que** le tissu (172) est non tissé.

7. Dispositif (170) selon la revendication 6, **caractérisé en ce que** le tissu non tissé est un mélange polypropylène-viscose.

8. Dispositif (170) selon l'une quelconque des revendications 5, 6 ou 7, **caractérisé en ce que** le tissu absorbant (172) est lié au matériau à déformation plastique (171), le matériau à déformation plastique (171) étant forcé parmi les fibres du tissu (172) pour former une liaison.

9. Dispositif de distribution (180) selon l'une quelconque des revendications 1 à 8, **caractérisé en ce que** le dispositif (180) est marqué par un ou plusieurs symboles visibles (181), et une couche de revêtement (182) est appliquée sur le ou les symboles (181).

10. Dispositif (180) selon la revendication 9, **caractérisé en ce que** la couche de revêtement (182) est constituée du même matériau que le matériau à déformation plastique, de sorte que le symbole (181) soit incorporé dans le matériau à déformation plastique.

11. Dispositif de distribution (170, 180) selon l'une quelconque des revendications précédentes, **caractérisé en ce que** le produit de soin bucco-dentaire est un produit de blanchissement des dents.

12. Dispositif de distribution (170, 180) selon l'une quelconque des revendications précédentes, **caractérisé en ce que** le produit de soin bucco-dentaire se présente sous la forme d'un gel.

13. Dispositif de distribution (170, 180) selon la revendication 12, **caractérisé en ce que** le produit de soin bucco-dentaire est un gel contenant du peroxyde.

14. Dispositif (170, 180) selon l'une quelconque des revendications précédentes, **caractérisé par** une forme allongée d'une longueur suffisante qui, une fois placée contre la surface avant des dents d'un utilisateur, s'étend à travers une pluralité de dents, et d'une largeur suffisante qui, une fois placée contre les dents de l'utilisateur, s'étend depuis la ligne gingivale au moins jusqu'aux couronnes des dents avant éloignées de la ligne gingivale.

15. Dispositif (170, 180) selon la revendication 14, **caractérisé en ce que** la largeur est telle que, dans un état déplié, la bande présente une largeur dépliée supérieure à la hauteur des dents de la ligne gingivale à la couronne, et au moins une partie de la bande peut être pliée sur un axe de pliage sensiblement longitudinal, de manière à se courber ou se replier sur les couronnes et à venir en contact avec les couronnes et les surfaces arrière des dents de l'utilisateur.

16. Dispositif (200, 210) selon la revendication 14 ou 15, **caractérisé en ce qu'**il est sensiblement rectangulaire avec des côtés longs incurvés de manière concave.

17. Dispositif (190, 220, 230, 240) selon la revendication 14 ou 15, **caractérisé en ce qu'**il est sensiblement rectangulaire avec des côtés longs courbés de manière convexe, ou d'une forme globalement rectangulaire mais avec un côté long incurvé de manière concave ou une indentation concave dans un côté long.

18. Dispositif selon la revendication 16 ou 17, **caractérisé par** une languette (201, 211) s'étendant depuis un côté long.

19. Procédé de réalisation d'un dispositif (170, 180) selon l'une quelconque des revendications 1 à 18, **caractérisé par** les étapes consistant à amener le matériau absorbant (172) en contact avec la surface de la bande (171) et à appliquer une pression, puis à appliquer le produit de soin bucco-dentaire sur le matériau absorbant (172).

20. Procédé selon la revendication 19, **caractérisé par** les étapes consistant à fournir une cire dentaire sous la forme d'une feuille (171), à imprimer un symbole visible (181) sur une première surface (171B) de la feuille (171) du matériau de cire, à laminer une seconde feuille de revêtement (182) du matériau de cire sur la première surface de la feuille, à intercaler le symbole imprimé entre les première et seconde feuilles, à dimensionner les feuilles laminées selon une épaisseur appropriée en les comprimant, à fixer la couche de tissu absorbant (172) sur la bande de cire laminée, à couper la feuille de matériau laminé selon la forme souhaitée, et à appliquer le produit de soin bucco-dentaire sur la couche de matériau absorbant.
